# EUROPEAN PATENT SPECIFICATION

(11) **EP 1 480 624 B1**
(45) Date of publication and mention of the grant of the patent: **29.11.2006**
(21) Application number: 03737712.4
(22) Date of filing: 06.02.2003
(51) Int. Cl.: A61K 9/34

(54) **PHARMACEUTICAL TABLET**
PHARMAZEUTISCHE TABLETTE
COMPRIME PHARMACEUTIQUE

(30) Priority: 07.02.2002 US 355705 P
(43) Date of publication of application: 01.12.2004
(73) Proprietor: Pharmacia Corporation, St. Louis, Missouri 63141 (US)
(72) Inventor: MARTINO, Alice, C., Kalamazoo, MI 49009 (US); NOACK, Robert, M., Grand Rapids, MI 49506 (US); PIERMAN, Steven, A., Portage, MI 49002 (US)
(74) Representative: Rutt, Jason Edward
(86) International application number: PCT/US2003/003837
(87) International publication number: WO 2003/066030

(56) References cited:
- WO-A-01/26634
- WO-A-94/18953
- US-A- 6 113 945

## Description

### FIELD OF THE INVENTION

The present invention relates to orally deliverable pharmaceutical dosage forms, more particularly tablets.

### BACKGROUND OF THE INVENTION

Tablets are the most common and convenient pharmaceutical dosage form for oral administration of medications. It is an important functional attribute of a tablet that it be readily identifiable by appearance, including by size, shape, surface texture, markings and color. Such ready identifiability is important in minimizing dispensing errors and in enabling patients on multiple medication to distinguish among the drugs they take at different times or frequencies. A vast array of drugs are now formulated as tablets, many of these drugs being formulated at different dosage strengths, and it is therefore becoming ever more important, yet ever more diffcult to insure, that any new tablet has unique appearance.

In particular, the range of readily distinguishable colors available to the formulator of tablets is rather limited. To some extent this problem has been alleviated by use of particolored, bicolored or multicolored tablets. However, a need remains in the art for tablets having unique surface color patterns.

In the case of coated tablets, color can be an attribute of the surface coating. For example, a range of opaque pigments are available for incorporation in tablet coating compositions such as those based on ethylcellulose; these pigments tend to impart a solid white or colored appearance to a coated tablet. Alternatively, the surface coating can be clear and transparent, and the color of the coated tablet is controlled by the color of the tablet core underlying the coating.

International Patent Publication No. WO 01/26634 discloses tablets with a gellan gum coating. The gellan gum aqueous composition may optionally contain a suitable color or colorants.

U.S. Patent No. 6,326,028 to Nivaggioli *et al* discloses a tablet coating comprising gellan gum. Such a coating is said to be useful for tablets to be taken orally, and to confer benefits in appearance, identification, mouth feel, reduced dust, stability, color and/or swallowability.

International Patent Publication No. WO 01/10406. discloses compositions said to be suitable for a wide range of routes of administration of sildenafil citrate, including buccal and sublingual routes. Preferred compositions disclosed are said to comprise a solution, gel, semisolid, suspension, metered dose device, transdermal patch or film. It is indicated that such compositions can include a gelling system, for example gellan gum 0.5% to 10%.

International Patent Publication No. WO 02/05820 discloses film dosage forms comprising sildenafil citrate. These dosage forms are prepared by mixing a solid dispersion of sildenafil citrate and a water-soluble sugar with a hydrocolloid and optionally other ingredients, and are said, upon placement on a mucosal surface, to form a coating that subsequently disintegrates and dissolves to release sildenafil. Gellan sodium salt is listed among hydrocolloids said to be useful in such film dosage forms.

U.S. Patent No. 6,291,506 to Levin discloses that the ophthalmic drug carvedilol can be formulated for ocular administration by suspending it in an agent such as gellan gum that will increase corneal contact time with the drug. Other possible delivery modes for the drug are contemplated therein. A claim is included to a method wherein the drug is delivered by a selection of routes including sublingually.

### SUMMARY OF THE INVENTION

There is now provided a pharmaceutical tablet comprising a core and a coating adherent thereto, wherein (a) the core comprises solid particles of a water-soluble dye distributed in a matrix, and (b) the coating comprises gellan gum.

The tablet is particularly suitable for peroral or intraoral administration, for example for delivery of a drug contained in the core of the tablet to a subject, illustratively a human subject. The term "peroral" herein refers to administration via the mouth involving swallowing of the tablet without substantial prior disintegration of the tablet in the mouth, so that absorption of the drug typically occurs in the gastrointestinal tract. The term "intraoral" herein refers to administration by placement of the tablet in the mouth of the subject, where the tablet disintegrates and/or dissolves, so that absorption of the drug typically occurs at least in part via the oral mucosa. For intraoral administration, the tablet can be placed in or on any part of the mouth, but placement of the tablet in the sublingual or buccal spaces is preferred.

The tablet can alternatively be dissolved or dispersed in a liquid vehicle, preferably water, and swallowed as a draft.

Tablets of the invention have an unusual speckled appearance. Without being bound by theory, it is believed that during the process of coating the core with an aqueous coating composition comprising gellan gum, dye particles in contact with the coating composition partially dissolve, causing color to "bleed" into the coating at the locus of each such particle. The color then becomes fixed as the coating dries. The speckled pattern is accentuated and rendered even more attractive or elegant by a high gloss surface texture contributed by the gellan gum. As described more fully hereinbelow, many variants of the speckled pattern characteristic of tablets of the invention are possible and practicable, adding a new option to the formulator seeking to prepare a readily identifiable tablet.

As a further advantage, the speckled pattern of tablets of the invention can obscure any small areas of discoloration that can sometimes result from variation in process conditions.

Other features, advantages and benefits of the invention will be apparent from the description that follows.

### DETAILED DESCRIPTION OF THE INVENTION

A tablet of the invention can be a placebo tablet, *i.e.,* containing no drug or other active agent in the core thereof. Preferably a tablet ofthe invention contains in the core a therapeutically and/or prophylactically useful amount of a drug, more preferably a drug that is advantageously delivered by peroral or intraoral admmistration.

For example, a drug present in the core of a tablet of the invention can be selected from the following illustrative classes: ACE inhibitors; α-adrenergic agonists; β-adrenergic agonists; α-adrenergic blockers; β-adrenergic blockers (beta blockers); alcohol deterrents; aldose reductase inhibitors; aldosterone antagonists; amino acids; anabolics; analgesics (both narcotic and non-narcotic); anesthetics; anorexics; antacids; anthelmintics; antiacne agents; antiallergics; antiandrogens; antianginal agents; antianxiety agents; antiarrythmics; antiasthmatics; antibacterial agents and antibiotics; antialopecia and antibaldness agents; antiamebics; antibodies; anticholinergic drugs; anticoagulants and blood thinners; anticolitis drugs; anticonvulsants; anticystitis drugs; antidepressants; antidiabetic agents; antidiarrheals; antidiuretics; antidotes; antiemetics; antiestrogens; antiflatulents; antifungal agents; antigens; antiglaucoma agents; antihistaminics; antihyperactives; antihyperlipoproteinemics; antihypertensives; antihyperthyroid agents; antihypotensives; antihypothyroid agents; anti-infectives; antiinflammatories (both steroidal and nonsteroidal); antimalarial agents; antimigraine agents; antineoplastics; antiobesity agents; antiparkinsonian agents and antidyskinetics; antipneumonia agents; antiprotozoal agents; antipruritics; antipsoriatics; antipsychotics; antipyretics; antirheumatics; antisecretory agents; anti-shock medications; antispasmodics; antithrombotics; antitumor agents; antitussives; antiulceratives; antiviral agents; anxiolytics; bactericidins; bone densifiers; bronchodilators; calcium channel blockers; carbonic anhydrase inhibitors; cardiotonics and heart stimulants; chemotherapeutics; choleretics; cholinergics; chronic fatigue syndrome medications; CNS stimulants; coagulants; contraceptives; cystic fibrosis medications; decongestants; diuretics; dopamine receptor agonists; dopamine receptor antagonists; enzymes; estrogens; expectorants; gastric hyperactivity medications; glucocorticoids; hemostatics; HMG CoA reductase inhibitors; hormones; hypnotics; immunomodulators; immunosuppressants; laxatives; medicaments for oral and periodontal diseases; miotics; monoamine oxidase inhibitors; mucolytics; multiple sclerosis medications; muscle relaxants; mydriatics; narcotic antagonists; NMDA receptor antagonists; oligonucleotides; ophthalmic drugs; oxytocics; peptides, polypeptides and proteins; polysaccharides; progestogens; prostaglandins; protease inhibitors; respiratory stimulants; sedatives; serotonin uptake inhibitors; sex hormones including androgens; smoking cessation drugs; smooth muscle relaxants; smooth muscle stimulants; thrombolytics; tranquilizers; urinary acidifiers; urinary incontinence medications; vasodilators; vasoprotectants; and combinations thereof.

It will be understood that any reference herein to a particular drug compound includes tautomers, stereoisomers, salts and prodrugs of that compound and is not specific to any one solid state form of the drug.

In one embodiment a drug contained in the core of the tablet is a smoking cessation drug, for example nicotine, a nicotine metabolite or a non-nicotine aid to smoking cessation such as bupropion or ibogaine.

Illustratively, a smoking cessation drug can be selected from nicotine and metabolites thereof (e.g., cotinine, norcotinine, nornicotine, nicotine N-oxide, cotinine N-oxide, 3-hydroxycotinine and 5-hydroxycotinine), ibogaine, bupropion and metabolites thereof (e.g., the erythro- and threo-amino alcohols of bupropion, the erythro-amino diol of bupropion and hydroxybupropion), lobeline, selegiline, risperidone and its 9-hydroxy metabolite, desmethylselegiline, substituted pyridine derivatives (*e.g*., 1-[(6-chloro-3-pyridinyl)methyl]-2-imidazolidine, 1-[(6-chloro-3-pyridinyl)methyl]-2-imidazothiazole and analogs thereof), methcamylamine, desipramine, fluoxetine, ropinirole, trimethaphan, trimethaphan camsylate, doxepin, 2-(3-chlorophenyl)-3,5,5-trimethyl-2-morpholinol, anxiolytics (e.g., isovaleramide), γ-vinyl GABA (GVG), epibatidine and derivatives thereof, 7-azabicyclo-[2.2.1]-heptane and -heptene compounds, naltrexone, nalmefene, ketamine, hexamethonium, pentolmium, dihydro-β-erythroidine, erysodine, d-tubocurarine, pempidine, chlorisondamine, amantadine, hetero-oxy alkanamines, benzylidene- and cinnamylidene-anabasines, azaindole-ethylamine derivatives, N-(pyridinylmethyl)-heterocyclylideneamines and NK-1 receptor antagonists (e.g., 9-bromo-1,2,3,4,5,6-hexahydro-1,5-methano-pyrido[1,2-a][1,5]diazocin-8-one).

In another embodiment a drug contained in the core of the tablet is an antibacterial drug. Illustratively such a drug can be an antibiotic, for example an aminoglycoside, amphenicol, ansamycin, carbapenem, cephalosporin, cephamycin, monobactam, oxacephem, penicillin, lincosamide, macrolide, polypeptide or tetracycline; or a synthetic antibacterial, for example a 2,4-diarninopyrimidine, nitrofuran, oxazolidinone, quinolone or analog thereof, sulfonamide or sulfone. Presently preferred antibacterials include the following illustrative examples: amikacin, azithromycin, cefixime, cefoperazone, cefotaxime, ceftazidime, ceftizoxime, ceftriaxone, chloramphenicol, ciprofloxacin, clindamycin, colistin, domeclocycline, doxycycline, erythromycin, gentamicin, lincomycin, linezolid, mafenide, methacycline, minocycline, neomycin, norfloxacin, ofloxacin, oxytetracycline, pirlimycin, polymyxin B, pyrimethamine, silver sulfadiazine, sulfacetamide, sulfisoxazole, tetracycline, tobramycin, trimethoprim and combinations thereof. In one embodiment an antibacterial drug present in the core of the tablet is an oxazolidinone, for example selected from (*S*)-N-[[3-[3-fluoro-4-[4-(hydroxyacetyl)-1-piperazinyl]phenyl]-2-oxo-5-oxazolidinyl]methyl]acetamide (eperezolid), (*S*)-N-[[3-[3-fluoro-4-[4-(morpholinyl)phenyl]-2-oxo-5-oxazolidinyl]methyl]acetamide (linezolid), N-[(5*S*)-3-[3-fluoro-4-[4-(2-fluoroethyl)-3-oxo-1-piperazinyl]phenyl-2-oxo-5-oxazohdinyl]methyl]acetamide, (*S*)-N-[[3-[5-(3-pyridyl)thiophen-2-yl]-2-oxo-5-oxazolidinyl]methyl]acetamide, and (*S*)-N-[[3-[5-(4-pyridyl)pyrid-2-yl]-2-oxo-5-oxazolidinyl]methyl]acetamide hydrochloride.

In another embodiment a drug contained in the core of the tablet is an antimigraine agent. Illustratively such an agent is an alk-ylxanthine, for example caffeine; a dopamine D₂ receptor agonist, for example alpiropride or lisuride; a GABA_{A} receptor modulator, for example ganaxolone; a 5-hydroxytriptamine (5-HT) receptor agonist, for example almotriptan, eletriptan, frovatriptan, naratriptan, rizatriptan, sumatriptan or zolmitriptan; ergot or a derivative thereof, for example ergotamine or dihydroergotamine; or a vasomodulator, for example dotarizine, fonazine or lomerizine.

In another embodiment a drug contained in the core of the tablet is useful in treating or preventing an ophthalmic disorder.

Illustratively such an ophthalmic drug can be an antibacterial, for example selected from the classes listed above.

Alternatively or in addition, such an ophthalmic drug can illustratively be an antiglaucoma or intraocular pressure lowering agent, such as (a) an α-adrenergic agonist or sympathomimetic, *e.g.,* adrenolone, apraclonidine, brimonidine or dipivefrin; (b) a β-adrenergic blocker, *e.g.,* acebutolol, adaprolol, alprenolol, atenolol, betaxolol, bufetolol, bufuralol, bunitrolol, bunolol, bupranolol, carteolol, carveddol, cetamolol, dexpropanolol, labetalol, levobunolol, metipranolol, metoprolol, nadolol, nifenalol, oxyprenolol, penbutolol, pindolol, practolol, pronethalol, propranolol, sotalol, timolol, tolamolol, toliprolol or vaninolol; (c) a carbonic anhydrase inhibitor, *e.g*., acetazolamide or dorzolamide; or (d) a prostaglandin or analog thereof, *e.g*., PGF_{2α} analogs such as bimatoprost, latanoprost, travoprost and unoprostone isopropyl.

Alternatively or in addition, such an ophthalmic drug can illustratively be a miotic, e.g., carbachol, physostigmine or pilocarpine.

Alternatively or in addition, such an ophthalmic drug can illustratively be an anti-inflammatory agent, for example an NSAID, more preferably a selective COX-2 inhibitory drug, for example selected from those listed below.

In another embodiment a drug contained in the core of the tablet is an analgesic, antipyretic or anti-inflammatory agent, *e.g*., aceclofenac, acemetacin, e-acetamidocaproic acid, acetaminophen, acetaminosalol, acetanilide, acetylsalicylic acid (aspirin), *S*-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, bezitramide, α-bisabolol, bromfenac, *p*-bromoacetanilide, 5-bromosalicylic acid acetate, bromosaligenin, bucetin, bucloxic acid, bucolome, budesonide, bufexamac, bumadizon, buprenorphine, butacetin, butibufen, butophanol, carbamazepine, carbiphene, carprofen, carsalam, celecoxib, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, deflazacort, desomorphine, desonide, desoximetasone, dexamethasone, dexoxadrol, dextromoramide, dezocine, diampromide, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, etoricoxib, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocortin butyl, fluocortolone, fluoresone, fluorometholone, fluperolone, flupirtine, fluprednidene, fluprednisolone, fluproquazone, flurandrenolide, flurbiprofen, formocortal, fosfosal, gentisic acid, glafenine, glucametacin, glycol salicylate, guaiazulene, halcinonide, halometasone, haloprednone, hydrocodone, hydrocortamate, hydrocortisone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p*-lactophenetide, lefetamine, levorphanol, lofentanil, lonazolac, lornoxicam, loxoprofen, lysine acetylsalicylate, mazipredone, meclofenamic acid, medrysone, mefenamic acid, meperidine, meprednisone, meptazinol, mesalamine, metazocine, methadone, methotrimeprazine, methylprednisolone, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paramethasone, paranyline, parecoxib, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, prednicarbate, prednisolone, prednisone, prednival, prednylidene, proglumetacin, proheptazine, promedol, propacetamol, propiram, propoxyphene, propyphenazone, proquazone, protizinic acid, proxazole, ramifenazone, remifentanil, rimazolium metilsulfate, rofecoxib, salacetamide, salicin, salicylamide, salicylamide o-acetic acid, salicylic acid, salicylsulfuric acid, salsalate, salverine, simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, tropesin, valdecoxib, viminol, xenbucin, ximoprofen, zaltoprofen or zomepirac.

In a particular embodiment such a drug is a selective COX-2 inhibitory drug, for example a compound of formula (I): 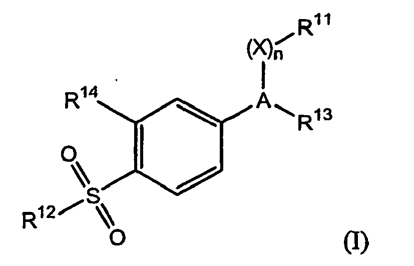 or a prodrug thereof or a pharmaceutically acceptable salt thereof, wherein:
- A: is a substituent selected from partially unsaturated or unsaturated heterocyclyl and partially unsaturated or unsaturated carbocyclic rings, preferably a heterocyclyl group selected from pyrazolyl, furanonyl, isoxazolyl, pyridinyl, cyclopentenonyl and pyridazinonyl groups;
- X: is O, S or CH₂;
- n: is 0 or 1;
- R¹¹: is at least one substituent selected from heterocyclyl, cycloalkyl, cycloalkenyl and aryl, and is optionally substituted at a substitutable position with one or more radicals selected from alkyl, haloalkyl, cyano, carboxyl, alkoxycarbonyl, hydroxyl, hydroxyalkyl, haloakoxy, amino, alkylamino, arylamino, nitro, alkoxyalkyl, alkylsulfinyl halo, alkoxy and alkylthio;
- R¹²: is methyl, amino or aminocarbonylalkyl;
- R¹³: is one or more radicals selected from hydrido, halo, alk-yl alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl and N-alkyl-N-arylaminosulfonyl, R¹³ being optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio; and
- R¹⁴: is selected from hydrido and halo.

In a preferred composition according to the present embodiment the selective COX-2 inhibitory drug is a compound having the formula (II): 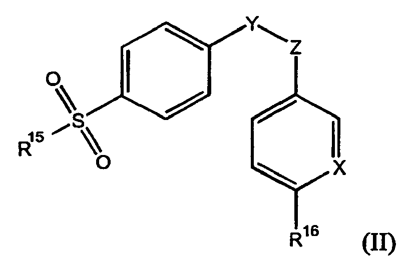 where R¹⁵ is a methyl, amino or imide group, R¹⁶ is hydrogen or a C₁₋₄ alkyl or alkoxy group, X is N or CR¹⁷ where R¹⁷ is hydrogen or halogen, and Y and Z are independently carbon or nitrogen atoms defining adjacent atoms of a five- to six-membered ring that is unsubstituted or substituted at one or more positions with oxo, halo, methyl or halomethyl groups. Preferred such five- to six-membered rings are cyclopentenone, furanone, methylpyrazole, isoxazole and pyridine rings substituted at no more than one position.

In another preferred composition according to the present embodiment the selective COX-2 inhibitory drug is a compound having the formula (III): 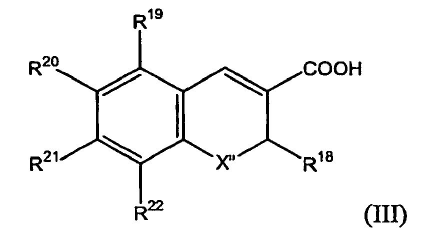 or a prodrug thereof or a pharmaceutically acceptable salt thereof, where X" is O, S or N-lower alkyl; R¹⁸ is lower haloalkyl; R¹⁹ is hydrogen or halogen; R²⁰ is hydrogen, halogen, lower alkyl, lower alkoxy or haloalkoxy, lower aralkylcarbonyl, lower dialkylaminosulfonyl, lower alkylaminosulfonyl, lower aralkylaminosulfonyl, lower heteroaralkylaminosulfonyl, or 5- or 6- membered nitrogen-containing heterocyclosulfonyl; and R²¹ and R²² are independently hydrogen, halogen, lower alkyl, lower alkoxy, or aryl.

A particularly useful compound of formula (III) is (S)-6,8-dichloro-2-(trifluoromethyl)-2H-1-benzopyran-3-carboxylic acid. In yet another preferred composition according to the present embodiment the selective COX-2 inhibitory drug is a 5-alkyl-2-arylaminophenylacetic acid or derivative thereof. Particularly useful compounds of this class are 5-methyl-2-(2'-chloro-6'-fluoroanilino)phenylacetic acid and pharmaceutically acceptable salts thereof.

Illustratively, celecoxib, deracoxib, valdecoxib, parecoxib, rofecoxib, etoricoxib, 2-(3,5-difluorophenyl)-3-[4-(methylsulfonyl)phenyl]-2-cyclopenten-1-one, (*S*)-6,8-dichloro-2-(trifluoromethyl)-2H-1-benzopyran-3-carboxylic acid, 2-(3,4-difluorophenyl)-4-(3-hydroxy-3-methyl-1-butyoxy)-5-[4-(methylsulfonyl)phenyl]-3-(2H)-pyridazinone and salts thereof are useful in compositions of the invention.

For example, the selective COX-2 inhibitory drug or prodrug thereof can be selected from celecoxib, valdecoxib, parecoxib, rofecoxib, etoricoxib, (*S*)-6,8-dichloro-2-(trifluoromethyl)-2H-1-benzopyran-3-carboxylic acid and salts thereof.

In another embodiment, a drug contained in the core of the tablet is useful in treatment and/or prevention of sexual dysfunction in male and/or female subjects. Such a drug can illustratively be (a) a phosphodiesterase type 5 (PDE5) inhibitor, e.g., sildenafil, tadalafil or vardenafil, (b) a cyclic GMP phosphodiesterase inhibitor, (c) a cyclic AMP activator, (d) an α-adrenergic antagonist, *e.g.,* phentolamine or yohimbine, or (e) a dopaminergic agonist, *e.g.,* apomorphine. Such a drug can be a compound of formula (V) below. Alternatively, a drug contained in the core of the tablet can be other than a drug useful in treatment and/or prevention of sexual dysfunction. As another alternative, a drug contained in the core of the tablet can be useful in treatment and/or prevention of sexual dysfunction but is other than a compound of formula (V) below.

In illustrative compositions a drug useful for example in treatment of Parkinson's disease or sexual dysfunction is present in the core of the tablet and is a compound of formula (V) 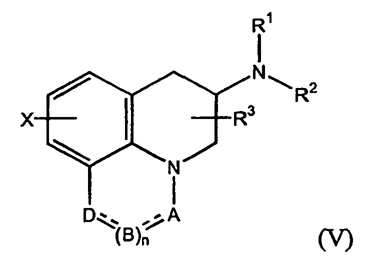 or a pharmaceutically acceptable salt thereof, wherein
- R¹,: R² and R³ are the same or different and are H, C₁₋₆ alkyl (optionally phenyl substituted), C₃₋₅ alkenyl or alkynyl or C₃₋₁₀ cycloalkyl, or where R³ is as above and R¹ and R² are cyclized with the attached N atom to form pyrrolidinyl, piperidinyl, morpholinyl, 4-methylpiperazinyl or imidazolyl groups;
- X: is H, F, Cl, Br, I, OH, C₁₋₆ alkyl or alkoxy, CN, carboxamide, carboxyl or (C₁₋₆ alkyl)carbonyl;
- A: is CH, CH₂, CHF, CHCl, CHBr, CHI, CHCH₃, C=O, C=S, CSCH₃, C=NH, CNH₂, CNHCH₃, CNHCOOCH₃, CNHCN, SO₂ or N;
- B: is CH, CH₂, CHF, CHCl, CHBr, CHI, C=O, N, NH or NCH₃, and n is 0 or 1; and
- D: is CH, CH₂, CHF, CHCl, CHBr, CHI, C=O, O, N, NH or NCH₃.
It is preferred that the compound of formula (V) or salt thereof is water-soluble.

Pharmaceutically acceptable salts of a compound of formula (V) include without restriction salts ofthe following acids: hydrochloric, hydrobromic, sulfuric, methanesulfonic, phosphoric, nitric, benzoic, citric, tartaric, fumaric and maleic acids, and mono- and dicarboxylic acids of formula CH₃-(CH₂)ₙ-COOH and HOOC-(CH₂)ₙ-COOH where n is 0 to 4, for example malonic acid.

Particularly preferred salts are the hydrochloride salt and the maleate, *i.e.,* (*Z*)-2-butenedioate, salt.

Compounds of formula (V) and their salts can be prepared by processes known *per se,* including processes described in patent literature cited herein. However, the present invention is not restricted by the process used to prepare the therapeutic agent.

Preferred compounds of formula (V) are those disclosed generically or specifically in U.S. Patent No. 5,273,975 to Moon *et al.* Especially preferred compounds are those of formula (VI) 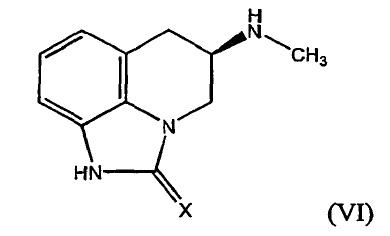 wherein X is O or S, and pharmaceutically acceptable salts thereof

Tablet cores useful according to the invention can be prepared by any suitable process known in the art. A core according to the invention comprises a matrix wherein are distributed solid particles of a water-soluble dye. Any suitable excipient or excipients can form the matrix. For peroral tablets the matrix typically comprises a diluent or carrier, for example lactose and/or starch, and can further comprise additional excipients such as binders, disintegrants, wetting agents, etc. For intraoral tablets the matrix typically comprises a water-soluble sugar, for example mannitol and/or maltose. If a drug is present in the core, the drug, too, is distributed in the matrix.

The ultimate appearance of the tablet depends in part upon the selection of water-soluble dye and the number and size of the solid particles of the dye. For example, relatively large particles will tend to produce a speckled pattern having larger blocks of color than will be produced by smaller particles; and a relatively large number of particles will tend to produce a speckled pattern where the color covers a greater portion of the tablet surface than will be produced by fewer particles. A more water-soluble dye will tend to produce larger and/or less discrete blocks of color than will be produced by a less water-soluble dye.

Optionally solid particles of more than one water-soluble dye can be present in the core, contributing a bicolored or multicolored speckled appearance to the tablet.

The core is coated with a coating composition comprising gellan gum, as more fully described below. The coating is typically present in an amount representing a weight gain of about 0.1 % to about 5%, but greater or lesser amounts can be used if desired. Preferably the gellan gum constitutes about 25% to 100%, more preferably about 50% to 100%, by weight of the coating.

Preferably the coating is an excipient coating. An "excipient coating" herein is a coating consisting, at least at the time of application ofthe coating to the core, only of excipient materials, *i.e.,* having substantially no drug present therein. It will be understood that during manufacture and storage some migration of a drug substance can potentially occur from the core to the coating of a tablet of the invention, but this is generally minimal. Thus a drug substance, if present in the tablet, is largely confined to the core where it is not commingled with gellan gum.

Any gellan gum can be used in the coating composition, but it is preferred to use a deacylated gellan gum such as that sold under the trademark Kelcogel^{™}. Optionally one or more additional gums and/or biopolymers, for example alginates, can be present in the coating composition.

The coating composition comprises a sprayable vehicle, preferably water, having dissolved or dispersed therein a gellan gum and optionally one or more additional excipients. Preferably the coating composition has a total solids concentration of about 1% to about 10% by weight, and a gellan gum concentration of about 1% to about 5% by weight.

Additional excipients present in the coating composition can include one or more buffering agents, typically at a concentration of about 0.03% to about 3% by weight; one or more plasticizers, typically at a concentration of about 0.03% to about 3% by weight; and/or one or more dispersing and/or emulsifying agents, typically at a concentration of about 0.03% to about 3% by weight. An example of a suitable buffering agent is sodium citrate. An example of a suitable plasticizer is propylene glycol. An example of a suitable dispersing or emulsifying agent is lecithin. Flavoring agents can also be included in the coating composition if desired.

The coating composition can be prepared by any suitable process involving dissolving the gellan gum and other, optional, excipients in the vehicle, preferably water. Order of addition is not critical. The water is preferably heated, for example to a temperature of about 55°C to about 85°C. Gellan gum and other excipients, if present, are added with stirring until all ingredients are homogeneously dispersed. The resulting coating liquid is preferably maintained at an elevated temperature during the stirring and subsequent spraying procedure.

Tablet cores to be coated are placed in a suitable coating apparatus, for example a coating pan, and are preferably preheated to a bed temperature of about 50°C to about 70°C. The coating liquid is sprayed on to the tablets under conditions that will be readily optimized by one of skill in the art. Spraying is continued until an amount of coating solution equivalent to a weight gain of about 0.1 % to about 5% has been applied. The resulting coated tablets are preferably cooled to ambient temperature, or about 20°C to about 35°C, prior to discharge from the coating pan.

Coating and cooling conditions can also affect the precise color pattern of the finished tablet. For example, if coating is done at lower temperatures and/or if cooling occurs slowly, so that the solid dye particles in the core are exposed to water for a longer period of time, a speckled pattern with larger blocks of color will typically - result.

An illustrative sublingual tablet of the invention containing as active agent a salt, e.g., the maleate salt, of sumanirole or (*R*)-5,6-dihydro-5-(methylamino)-4H-imidazo[4,5-*ij*]-quinoline-2(1H)-thione has a core having the following composition:

| | |
|---|---|
| active agent | 0.1-3% free base equivalent |
| mannitol | 50-90% |
| powdered sorbitol | 10-40% |
| hydroxypropylcellulose | 0-10% |
| xanthan gum | 0-5% |
| flavoring agent | 0-0.5% |
| water-soluble dye | 0-0.5% |
| colloidal silicon dioxide | 0-1% |
| magnesium stearate | 0.5-5% |

all percentages being by weight.

Another illustrative sublingual tablet of the invention containing as active agent a salt, *e.g*., the maleate salt, of sumanirole or (*R*)-5,6-dihydro-5-(methylamino)-4H-imidazo[4,5-*ij*]-quinoline-2(1H)-thione has a core having the following composition:

| | |
|---|---|
| active agent | 0.1-3% free base equivalent |
| lactose monohydrate | 50-85% |
| pregelatinized starch | 10-45% |
| xanthan gum | 0-5% |
| flavoring agent | 0-0.5% |
| water-soluble dye | 0-0.5% |
| colloidal silicon dioxide | 0-1% |
| magnesium stearate | 0.5-5% |

all percentages being by weight.

Yet another illustrative sublingual tablet of the invention containing as active agent a salt, e.g., the maleate salt, of sumanirole or (*R*)-5,6-dihydro-5-(methylamino)-4H-imidazo[4,5-*ij*]-quinoline-2(1H)-thione has a core having the following composition:

| | |
|---|---|
| active agent | 0.1-3% free base equivalent |
| microcrystalline cellulose | 30-70% |
| pregelatinized starch | 25-65% |
| croscarmellose sodium | 0-10% |
| xanthan gum | 0-5% |
| flavoring agent | 0-0.5% |
| water-soluble dye | 0-0.5% |
| colloidal silicon dioxide | 0-1% |
| magnesium stearate | 0.5-5% |

all percentages being by weight.

### EXAMPLES

The following examples illustrate aspects of the present invention but should not be construed as limitations. In these examples "compound Z" refers to (R)-5,6-dihydro-5-(methylamino)-4H-imidazo[4,5-*ij*]-quinoline-2(1H)-thione, maleate salt. All percentages are by weight unless otherwise indicated.

### Example 1

A sublingual tablet formulation was prepared having the following composition:

| | |
|---|---|
| compound Z | 1.11% |
| Avicel^{™} PH-101 (microcrystalline cellulose) | 46.71% |
| Starch 1500 of Colorcon (pregelatinized starch) | 44.00% |
| croscarmellose sodium NF | 5.00% |
| colloidal silicon dioxide NF | 0.50% |
| cinnamon flavor | 0.14% |
| mint flavor | 0.04% |
| dye (cherry shade #1632, Crompton & Knowles) | 0.50% |
| magnesium stearate | 2.00% |

Pregelatinized starch and dye were blended in a high-shear mixer for 2 minutes or until homogeneously mixed. The following ingredients were then individually layered over the resulting mixture in the high-shear mixer: compound Z; microcrystalline cellulose; colloidal silicon dioxide; croscarmellose sodium. Mixing in the high-shear mixer was resumed for a further 2 minutes. If the dye was not adequately dispersed throughout the resulting mixture, mixing continued in 1 minute increments until good dispersion of dye was observed. A small portion of the mixture was then removed and hand-mixed with magnesium stearate to form a magnesium stearate premix. This premix, together with the flavors, was added to the high-shear mixer and mixed for 1 minute to form a lubricated tablet stock.

The lubricated tablet stock was discharged from the high-shear mixer and stored in desiccated hermetically sealed containers until ready for tableting. Tablets were prepared by compression using 12/32 inch (approximately 9 mm) Plain/Plain tooling with slight curvature to the following specifications:

| | |
|---|---|
| tablet weight | 180 mg |
| hardness | 3-4 SCU |
| friability | <0.5% |

### Example 2

Sublingual tablets prepared as in Example 1 were coated with a gellan gum coating according to the following procedure.

A coating liquid having the following composition was prepared:

| | |
|---|---|
| gellan gum (Kelcogel^{™}) | 2.00% |
| sodium citrate | 0.13% |
| propylene glycol | 0.40% |
| lecithin | 0.20% |
| deionized water | 97.27% |

Deionized water was heated to 70°C. The other ingredients were added with stirring until all ingredients were homogeneously dispersed. The resulting coating liquid having a solids content of 2.73% was maintained at a temperature of 70°C during the stirring and subsequent spraying procedure.

Tablets of Example 1, in an amount of 700 g, were placed in a 12 inch (approximately 300 mm) coating pan and preheated to a bed temperature of 60°C. The coating liquid was sprayed on to the tablets under the following conditions:

| | |
|---|---|
| outlet air temperature | 50-60°C |
| pan speed | 16 rpm |
| air flow | 30-35 cfm (0.84-0.98 m³/minute) |
| atomizing air pressure | 10 psi (69 kPa) |
| peristaltic pump setting | 15-20 g/minute |

Spraying was continued until an amount of coating solution equivalent to a weight gain of 1.2% had been applied. The resulting coated tablets were cooled to 30°C prior to discharge from the coating pan.

The tablets had an attractive high gloss appearance with cherry red speckles.

### Example 3

A sublingual tablet formulation was prepared having the following composition:

| | |
|---|---|
| compound Z | 1.05% |
| mannitol, granular | 70.00% |
| sorbitol | 16.57% |
| hydroxypropylcellulose, type LH-11 | 7.00% |
| xanthan gum | 2.50% |
| colloidal silicon dioxide NF | 0.50% |
| cinnamon flavor | 0.14% |
| mint flavor | 0.04% |
| dye (cherry shade #1632, Crompton & Knowles) | 0.20% |
| magnesium stearate | 2.00% |

Mannitol and dye were blended in a high-shear mixer for 2 minutes or until homogeneously mixed. The following ingredients were then individually layered over the resulting mixture in the high-shear mixer: compound Z; sorbitol; hydroxypropylcellulose; xanthan gum; colloidal silicon dioxide. Mixing in the high-shear mixer was resumed for a further 2 minutes. If the dye was not adequately dispersed throughout the resulting mixture, mixing continued in 1 minute increments until good dispersion of dye was observed. A small portion of the mixture was then removed and hand-mixed with magnesium stearate to form a magnesium stearate premix. This premix, together with the flavors, was added to the high-shear mixer and mixed for 1 minute to form a lubricated tablet stock.

The lubricated tablet stock was discharged from the high-shear mixer and stored in desiccated hermetically sealed containers until ready for tableting. Tablets were prepared by compression using 12/32 inch (approximately 9 mm) Plain/Plain tooling with slight curvature to the following specifications:

| | |
|---|---|
| tablet weight | 190 mg |
| hardness | 3-4 SCU |
| friability | <0.5% |

### Example 4

Sublingual tablets prepared as in Example 3 were coated with a gellan gum coating according to the following procedure.

A coating liquid having the following composition was prepared:

| | |
|---|---|
| gellan gum (Kelcogel^{™}) | 2.00% |
| sodium citrate | 0.13% |
| propylene glycol | 0.40% |
| lecithin (Lipoid^{™} LS-100) | 0.20% |
| flavor | 0.30% |
| deionized water | 96.97% |

Deionized water was heated to 70°C. The other ingredients were added with stirring until all ingredients were homogeneously dispersed. The resulting coating liquid having a solids content of 3.03% was maintained at a temperature of 70°C during the stirring and subsequent spraying procedure.

Tablets of Example 1, in an amount of 700 g, were placed in a 12 inch (approximately 300 mm) coating pan and preheated to a bed temperature of 60°C. The coating liquid was sprayed on to the tablets under the following conditions:

| | |
|---|---|
| outlet air temperature | 50-60°C |
| pan speed | 16 rpm |
| air flow | 30-35 cfm (0.84-0.98 m³/minute) |
| atomizing air pressure | 10 psi (69 kPa) |
| peristaltic pump setting | 15-20 g/minute |

Spraying was continued until an amount of coating solution equivalent to a weight gain of 1.36% had been applied. The resulting coated tablets were cooled to 30°C prior to discharge from the coating pan.

The tablets had an attractive high gloss appearance with cherry red speckles.

### Example 5

A sublingual tablet formulation was prepared having the following composition:

| | |
|---|---|
| compound Z | 0.43% |
| Avicel^{™} PH-101 (microcrystalline cellulose) | 47.39% |
| Starch 1500 of Colorcon (pregelatinized starch) | 44.00% |
| croscarmellose sodium NF | 5.00% |
| colloidal silicon dioxide NF | 0.50% |
| cinnamon flavor | 0.14% |
| mint flavor | 0.04% |
| color (cherry shade #1632, Crompton & Knowles) | 0.50% |
| magnesium stearate | 2.00% |

Pregelatinized starch and color were blended in a high-shear mixer for 2 minutes or until homogeneously mixed. The following ingredients were then individually layered over the resulting mixture in the high-shear mixer: compound Z; microcrystalline cellulose; colloidal silicon dioxide; croscarmellose sodium Mixing in the high-shear mixer was resumed for a further 2 minutes. If the color was not adequately dispersed throughout the resulting mixture, mixing continued in 1 minute increments until good dispersion of color was observed. A small portion of the mixture was then removed and hand-mixed with magnesium stearate to form a magnesium stearate premix. This premix, together with the flavors, was hand screened through a #20 mesh pharmaceutical screen, then added to the high-shear mixer and mixed for 1 minute to form a lubricated tablet stock.

The lubricated tablet stock was discharged from the high-shear mixer and stored in desiccated hermetically sealed containers until ready for tableting. Tablets were prepared by compression using 12/32 inch (approximately 9 mm) Plain/Plain tooling with slight curvature to the following specifications:

| | |
|---|---|
| tablet weight | 180 mg |
| hardness | 3.5-4 SCU |
| friability | <0.8% |

### Example 6

Sublingual tablets prepared as in Example 5 were coated with a gellan gum coating according to the following procedure.

A coating liquid having the following composition was prepared:

| | |
|---|---|
| gellan gum (Kelcogel^{™}) | 2.00% |
| sodium citrate | 0.13% |
| propylene glycol | 0.40% |
| lecithin (Lipoid^{™} LS-100) | 0.20% |
| hot cinnamon flavor | 0.30% |
| deionized water | 96.97% |

Deionized water was heated to 70°C. The other ingredients were added with stirring until all ingredients were homogeneously dispersed. The resulting coating liquid having a solids content of 3.03% was maintained at a temperature of 70°C during the stirring and subsequent spraying procedure.

Tablets of Example 5, in an amount of 7000 g, were placed in a 24 inch (approximately 600 mm) coating pan and preheated to a bed temperature of 60°C. The coating liquid was sprayed on to the tablets under the following conditions:

| | |
|---|---|
| outlet air temperature | 48-55°C |
| pan speed | 10-14 rpm, preferably 14 rpm |
| air flow | 300-400 cfm (8.5-11.3 m³/minute) |
| atomizing air pressure | 20-35 psi (138-242 kPa), preferably about 20 psi |
| peristaltic pump setting | 15-40 g/minute/gun (2 gun spray system), preferably 30-40 g/minute/gun |
| tablet bed temp | 37-50°C, preferably about 40°C |

Spraying was continued until an amount of coating solution equivalent to a weight gain of 2.04% had been applied. The resulting coated tablets were cooled to 30°C prior to discharge from the coating pan.

The tablets had an attractive high gloss appearance with cherry red speckles.

## Claims

1. A pharmaceutical tablet comprising a core and a coating adherent thereto, wherein (a) the core comprises solid particles of a water-soluble dye distributed in a matrix; and (b) the coating comprises gellan gum.

2. The tablet of Claim 1 having a speckled surface appearance.

3. The tablet of Claim 1 wherein the core comprises a drug in a therapeutically and/or prophylactically effective amount.

4. The tablet of Claim 3 wherein the drug is selected from the group consisting of ACE inhibitors; α-adrenergic agonists; β-adrenergic agonists; α-adrenergic blockers; β-adrenergic blockers; alcohol deterrents; aldose reductase inhibitors; aldosterone antagonists; amino acids; anabolics; analgesics (both narcotic-and non-narcotic); anesthetics; anorexics; antacids; anthelmintics; antiacne agents; antiallergics; antiandrogens; antianginal agents; antianxiety agents; antiarrythmics; antiasthmatics; antibacterial agents and antibiotics; antialopecia and antibaldness agents; antiamebics; antibodies; anticholinergic drugs; anticoagulants and blood thinners; anticolitis drugs; anticonvulsants; anticystitis drugs; antidepressants; antidiabetic agents; antidiarrheals; antidiuretics; antidotes; antiemetics; antiestrogens; antiflatulents; antifungal agents; antigens; antiglaucoma agents; antihistaminics; antihyperactives; antihyperlipoproteinemics; antihypertensives; antihyperthyroid agents; antihypotensives; antihypothyroid agents; anti-infectives; antiinflammatories (both steroidal and nonsteroidal); antimalarial agents; antimigraine agents; antineoplastics; antiobesity agents; antiparkinsonian agents and antidyskinetics; antipneumonia agents; antiprotozoal agents; antipruritics; antipsoriatics; antipsychotics; antipyretics; antirheumatics; antisecretory agents; anti-shock medications; antispasmodics; antithrombotics; antitumor agents; antitussives; antiulceratives; antiviral agents; anxiolytics; bactericidins; bone densifiers; bronchodilators; calcium channel blockers; carbonic anhydrase inhibitors; cardiotonics and heart stimulants; chemotherapeutics; choleretics; cholinergics; chronic fatigue syndrome medications; CNS stimulants; coagulants; contraceptives; cystic fibrosis medications; decongestants; diuretics; dopamine receptor agonists; dopamine receptor antagonists; enzymes; estrogens; expectorants; gastric hyperactivity medications; glucocorticoids; hemostatics; HMG CoA reductase inhibitors; hormones; hypnotics; immunomodulators; immunosuppressants; laxatives; medicaments for oral and periodontal diseases; miotics; monoamine oxidase inhibitors; mucolytics; multiple sclerosis medications; muscle relaxants; mydriatics; narcotic antagonists; NMDA receptor antagonists; oligonucleotides; ophthalmic drugs; oxytocics; peptides, polypeptides and proteins; polysaccharides; progestogens; prostaglandins; protease inhibitors; respiratory stimulants; sedatives; serotonin uptake inhibitors; sex hormones; smoking cessation drugs; smooth muscle relaxants; smooth muscle stimulants; thrombolytics; tranquilizers; urinary acidifiers; urinary incontinence medications; vasodilators; vasoprotectants; and combinations thereof.

5. The tablet of Claim 3 wherein the drug is a smoking cessation drug.

6. The tablet of Claim 3 wherein the drug is an antibacterial drug.

7. The tablet of Claim 3 wherein the drug is an antimigraine agent.

8. The tablet of Claim 3 wherein the drug is useful in treating or preventing an ophthalmic disorder.

9. The tablet of Claim 3 wherein the drug is an analgesic, antipyretic or anti-inflammatory agent.

10. The tablet of Claim 9 wherein the analgesic, antipyretic or anti-inflammatory agent is selected from the group consisting of aceclofenac, acemetacin, e-acetamidocaproic acid, acetaminophen, acetaminosalol, acetanilide, acetylsalicylic acid, *S*-adenosylmethionine, alclofenac, alclometasone, alfentanil, algestone, allylprodine, alminoprofen, aloxiprin, alphaprodine, aluminum bis (acetylsalicylate), amcinonide, amfenac, aminochlorthenoxazin, 3-amino-4- hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, bezitramide, α-bisabolol, bromfenac, *p*-bromoacetanilide, 5-bromosalicylic acid acetate, bromosaligenin, bucetin, bucloxic acid, bucolome, budesonide, bufexamac, bumadizon, buprenorphine, butacetin, butibufen, butophanol, carbamazepine, carbiphene, carprofen, carsalam, celecoxib, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, deflazacort, desomorphine, desonide, desoximetasone, dexamethasone, dexoxadrol, dextromoramide, dezocine, diampromide, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, etoricoxib, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocortin butyl, fluocortolone, fluoresone, fluorometholone, fluperolone, flupirtine, fluprednidene, fluprednisolone, fluproquazone, flurandrenolide, flurbiprofen, formocortal, fosfosal, gentisic acid, glafenine, glucametacin, glycol salicylate, guaiazulene, halcinonide, halometasone, haloprednone, hydrocodone, hydrocortamate, hydrocortisone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p-*lactophenetide, lefetamine, levorphanol, lofentanil, lonazolac, lornoxicam, loxoprofen, lysine acetylsalicylate, mazipredone, meclofenamic acid, medrysone, mefenamic acid, meperidine, meprednisone, meptazinol, mesalamine, metazocine, methadone, methotrimeprazine, methylprednisolone, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, Nicomorphine, Nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paramethasone, paranyline, parecoxib, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, prednicarbate, prednisolone, prednisone, prednival, prednylidene, proglumetacin, proheptazine, promedol, propacetamol, propiram, propoxyphene, propyphenazone, proquazone, protizinic acid, proxazole, ramifenazone, remifentanil, rimazolium metilsulfate, rofecoxib, salacetamide, salicin, salicylamide, salicylamide o-acetic acid, salicylic acid, salicylsulfuric acid, salsalate, salverine,-simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, tropesin, valdecoxib, viminol, xenbucin, ximoprofen, zaltoprofen and zomepirac.

11. The tablet of Claim 3 wherein the drug is an agent useful in treatment and/or prevention of sexual dysfunction.

12. The tablet of any previous claim that is suitable for peroral or intraoral administration.

13. The tablet of any previous claim wherein the coating is present in an amount representing a weight gain of 0.1 % to 5%.

14. The tablet of any previous claim wherein the gellan gum constitutes 25% to 100% by weight of the coating.

15. The tablet of any previous claim wherein the coating further comprises at least one additional excipient selected from the group consisting of buffering agents, plasticizers and dispersing and emulsifying agents.

16. The tablet of claim 1, wherein said coating is spray coated.

17. The tablet of claim 2, wherein said gellan gum is deacylated gellan gum.

18. The tablet of claim 4, wherein said drug is selected from the group consisting of antihistamines, antitussives, decongestants, and combinations thereof.

19. The tablet of claim 10, wherein said drug is selected from the group consisting of acetaminophen, acetylsalicylic acid, celecoxib and ibuprofen.

## Patentansprüche

1. Pharmazeutische Tablette, umfassend einen Kern und einen daran haftenden Überzug, wobei (a) der Kern feste Teilchen eines wasserlöslichen Farbstoffs, verteilt in einer Matrix, umfasst und (b) der Überzug Gellangummi umfasst.

2. Tablette nach Anspruch 1 mit einem gefleckten Aussehen der Oberfläche.

3. Tablette nach Anspruch 1, wobei der Kern ein Arzneimittel in einer therapeutisch und/oder prophylaktisch wirksamen Menge umfasst.

4. Tablette nach Anspruch 3, wobei das Arzneimittel aus der Gruppe, bestehend aus ACE-Hemmern, α-adrenergen Agonisten, β-adrenergen Agonisten, α-adrenergen Blockern, β-adrenergen Blockern, Alkoholdeterrentien, Aldosereduktasehemmern, Aldosteronantagonisten, Aminosäuren, Anabolika, Analgetika (sowohl narkotisch als auch nicht-narkotisch), Anästhetika, Appetitzüglern, Antiazida, Anthelmintika, Antiaknemitteln, Mitteln gegen Allergien, antiandrogen wirkenden Mitteln, antiangialen Mitteln, Mitteln gegen Angstzustände, Antiarrhythmika, Antiasthmatika, antibakteriellen Mitteln und Antibiotika, Mitteln gegen Alopezie und Haarausfall, Mitteln gegen Amöben, Antikörpern, anticholinergen Arzneimitteln, Antikoagulantien und Blutverdünnern, Anticolitis-Arzneimittel, Antikonvulsiva, Mitteln gegen Cystitis, Antidepressiva, antidiabetischen Mitteln, Mitteln gegen Diarrhoe, Antidiuretika, Antidote, Antiemetika, Antiöstrogenen, Antiflatulentien, Mitteln gegen Pilze, Antigenen, Antiglaukom-Mitteln, Antihistaminika, Mitteln gegen Hyperaktivität, Mitteln gegen Hyperlipoproteinämie, Mitteln gegen Bluthochdruck, Antihyperthyroid-Mitteln, antihypotensiven Mitteln, antihypothyroiden Mitteln, Mitteln gegen Infektionen, entzündungshemmenden Mitteln (sowohl steroidal als auch nicht-steroidal), Antimalaria-Mitteln, Antimigräne-Mitteln, Antineoplastika, Mitteln gegen Fettsucht, Mitteln gegen Parkinsonsche-Krankheit und Antidyskinetika, Mitteln gegen Lungenentzündung, antiprotozoalen Mitteln, Mitteln gegen Juckreiz, Antipsoriatika, Antipsychotika, Antipyretika, Antirheumatika, antisekretorischen Mitteln, Medikationen gegen Schock, Antispasmodika, Antithrombotika, Antitumor-Mitteln, Antitussiva, Mitteln gegen Geschwüre, antiviralen Mitteln, Anxiolytika, Bakterizidinen, Knochenverdichtungsmitteln, Bronchodilatoren, Calciumkanalblockern, Kohlensäureanhydrasehemmern, Kardiotonika und Herzstimulantien, Chemotherapeutika, choleretischen Mitteln, cholinergen Mitteln, Medikationen gegen chronisches Ermüdungssyndrom, ZNS-Stimulantien, Koagulantien, Kontrazeptiva, Medikationen gegen cystische Fibrose, Dekongestantien, Diuretika, Dopaminrezeptoragonisten, Dopaminrezeptorantagonisten, Enzymen, Östrogenen, Expektorantien, Medikationen gegen Magenhyperaktivität, Glucocorticoiden, Hämostatika, HMG-CoA-Reduktasehemmern, Hormonen, Hypnotika, Immunomodulatoren, immunosuppressiven Mitteln, Laxativa, Medikamenten gegen Mund und periodontale Erkrankungen, Miotika, Monoaminoxidasehemmern, Mucolytika, Medikationen gegen multiple Sklerose, Muskelrelaxantien, Mydriatika, Antagonisten für Narkotika, NMDA-Rezeptorantagonisten, Oligonukleotiden, Arzneimitteln für die Augenheilkunde, Wehenmitteln, Peptiden, Polypeptiden und Proteinen, Polysacchariden, Progestogenen, Prostaglandinen, Proteasehemmern, Stimulantien für das respiratorische System, Sedativa, Serotoninaufnahmehemmern, Sexualhormonen, Arzneimitteln zur Entwöhnung von Rauchern, Relaxantien für die glatte Muskulatur, Stimulantien für die glatte Muskulator, thrombolytisch wirkenden Arzneimitteln, Tranquilizern, Mitteln zur Ansäuerung von Urin, Medikationen bei Harninkontinenz, Vasodilatoren, Vasoprotektanten und Kombinationen davon, ausgewählt worden ist.

5. Tablette nach Anspruch 3, wobei das Arzneimittel ein Arzneimittel zur Entwöhnung von Rauchern ist.

6. Tablette nach Anspruch 3, wobei das Arzneimittel ein antibakterielles Arzneimittel ist.

7. Tablette nach Anspruch 3, wobei das Arzneimittel ein Antimigränemittel ist.

8. Tablette nach Anspruch 3, wobei das Arzneimittel zur Behandlung oder Prävention von ophthalmischen Störungen geeignet ist.

9. Tablette nach Anspruch 3, wobei das Arzneimittel ein analgetisches, antipyretisches oder entzündungshemmendes Mittel ist.

10. Tablette nach Anspruch 9, wobei das analgetische, das antipyretische oder das entzündungshemmende Mittel aus der Gruppe, bestehend aus Aceclofenac, Acemetacin, e-Acetamidocapronsäure, Acetaminophen, Acetaminosalol, Acetanilid, Acetylsalicylsäure, S-Adenosylmethionin, Alclofenac, Alclometason, Alfentanil, Algeston, Allylprodin, Alminoprofen, Aloxipirin, Alphaprodin, Aluminiumbis(acetylsalicylat), Amcinonid, Amfenac, Aminochlorthenoxazin, 3-Amino-4-hydroxybuttersäure, 2-Amino-4-picolin, Aminopropylon, Aminopyrin, Amixetrin, Ammmoniumsalicylat, Ampiroxicam, Amtolmetinguacil, Anileridin, Antipyrin, Antrafenin, Apazon, Beclomethason, Bendazac, Benorylat, Benoxaprofen, Benzpiperylon, Benzydamin, Benzylmorphin, Bermoprofen, Betamethason, Bezitramid, α-Bisabolol, Bromfenac, p-Bromacetanilid, 5-Bromsalicylsäureacetat, Bromsaligenin, Bucetin, Bucloxinsäure, Bucolom, Budenosid, Bufexamac, Bumadizon, Buprenorphin, Butacetin, Butibufen, Butophanol, Carbamazepin, Carbiphen, Carprofen, Carsalam, Celecoxib, Chlorbutanol, Chlorprednison, Chlorthenoxazin, Cholinsalicylat, Cinchophen, Cinmetacin, Ciramodol, Clidanac, Clobetasol, Clocortolon, Clometacin, Clonitazen, Clonixin, Clopirac, Cloprednol, Nelkenöl bzw. Eugenol, Codein, Codeinmethylbromid, Codeinphosphat, Codeinsulfat, Cortison, Cortivazol, Cropropamid, Crotethamid, Deflazacort, Desomorphin, Desonid, Desoximetason, Dexamethason, Dexoxadrol, Dextromoramid, Dezocin, Diampromid, Diclofenac, Difenamizol, Difenpiramid, Diflorason, Diflucortolon, Diflunisal, Difluprednat, Dihydrocodein, Dihydrocodeinonenolacetat, Dihydromorphin, Dihydroxyaluminiumacetylsalicylat, Dimenoxadol, Dimepheptanol, Dimethylthiambuten, Dioxaphetylbutyrat, Dipipanon, Diprocetyl, Dipyron, Ditazol, Droxicam, Emorfazon, Enfenaminsäure, Enoxolon, Epirizol, Eptazocin, Etersalat, Ethenzamid, Ethoheptazin, Ethoxazen, Ethylmethylthiambuten, Ethylmorphin, Etodolac, Etofenamat, Etonitazen, Etoricoxib, Eugenol, Felbinac, Fenbufen, Fenclozinsäure, Fendosal, Fenoprofen, Fentanyl, Fentiazac, Fepradinol, Feprazon, Floctafenin, Fluazacort, Flucloronid, Flufenaminsäure, Flumethason, Flunisolid, Flunixin, Flunoxaprofen, Fluocinolonacetonid, Fluocinonid, Fluocortinbutyl, Fluocortolon, Fluoreson, Fluorometholon, Fluperolon, Flupirtin, Flupredniden, Fluprednisolon, Fluproquazon, Flurandrenolid, Flurbiprofen, Formocortal, Fosfosal, Gentisinsäure, Glafenin, Glucametacin, Glykolsalicylat, Guaiazulen, Halcinonid, Halometason, Haloprednon, Hydrocodon, Hydrocortamat, Hydrocortison, Hydromorphon, Hydroxypethidin, Ibufenac, Ibuprofen, Ibuproxam, Imidazolsalicylat, Indomethacin, Indoprofen, Isofezolac, Isoladol, Isomethadon, Isonixin, Isoxepac, Isoxicam, Ketobemidon, Ketoprofen, Ketorolac, p-Lactophenetid, Lefetamin, Levorphanol, Lofentanil, Lonozolac, Lonoxicam, Loxoprofen, Lysinacetylsalicylat, Mazipredon, Meclofenaminsäure, Medryson, Mefenaminsäure, Meperidin, Meprednison, Meptazinol, Mesalamin, Metazocin, Methadon, Methotrimeprazin, Methylprednisolon, Metiazinsäure, Metofolin, Metopon, Mofebutazon, Mofezolac, Morazon, Morphin, Morphinhydrochlorid, Morphinsulfat, Morpholinsalicylat, Myrophin, Nabumeton, Nalbuphin, 1-Naphthylsalicylat, Naproxen, Narcein, Nefopam, Nicomorphin, Nifenazon, Nifluminsäure, Nimesulid, 5'-Nitro-2'-propoxyacetanilid, Norlevorphanol, Normethadon, Normorphin, Norpipanon, Olsalazin, Opium, Oxaceprol, Oxametacin, Oxaprozin, Oxycodon, Oxymorphon, Oxyphenbutazon, Papaveretum, Paramethason, Paranylin, Parecoxib, Parsalmid, Pentazocin, Perisoxal, Phenacetin, Phenadoxon, Phenazocin, Phenazopyridinhydrochlorid, Phenocol, Phenoperidin, Phenopyrazon, Phenylacetylsalicylat, Phenylbutazon, Phenylsalicylat, Phenyramidol, Piketoprofen, Piminodin, Pipebuzon, Piperylon, Piprofen, Pirazolac, Piritramid, Piroxicam, Pranoprofen, Prednicarbat, Prednisolon, Prednison, Prednival, Prednyliden, Proglumetacin, Proheptazin, Promedol, Propacetamol, Propiram, Propoxyphen, Propyphenazon, Proquazon, Protizinsäure, Proxazol, Ramifenazon, Remifentanil, Rimazoliummetilsulfat, Rofecoxib, Salacetamid, Salicin, Salicylamid, Salicylamido-essigsäure, Salicylsäure, Salicylschwefelsäure, Salsalat, Salverin, Simetrid, Sufentanil, Sulfasalazin, Sulindac, Superoxiddismutase, Suprofen, Suxibuzon, Talniflumat, Tenidap, Tenoxicam, Terofenamat, Tetrandrin, Thiazolinbutazon, Tiaprofensäure, Tiaramid, Tilidin, Tinoridin, Tixocortol, Tolfenaminsäure, Tolmetin, Tramadol, Triamcinolon, Tropesin, Valdecoxib, Viminol, Xenbucin, Ximoprofen, Zaltoprofen und Zomepirac, ausgewählt worden ist.

11. Tablette nach Anspruch 3, wobei das Arzneimittel ein Mittel ist, das für die Behandlung und/oder Prävention von Sexualdysfunktionen geeignet ist.

12. Tablette nach einem der vorstehenden Ansprüche, geeignet für die perorale oder intraorale Verabreichung

13. Tablette nach einem der vorstehenden Ansprüche, wobei der Überzug in einer Menge vorhanden ist, die eine Gewichtszunahme von 0,1% bis 5% darstellt.

14. Tablette nach einem der vorstehenden Ansprüche, wobei der Anteil des Gellangummis 25 bis 100 Gew.-% des Überzugs beträgt.

15. Tablette nach einem der vorstehenden Ansprüche, wobei der Überzug weiterhin mindestens ein zusätzliches Exzipiens, ausgewählt aus der Gruppe, bestehend aus Puffermitteln, Weichmachern und Dispergierungs- und Emulgierungsmitteln, umfasst.

16. Tablette nach Anspruch 1, wobei der Überzug Sprühbeschichtet wird.

17. Tablette nach Anspruch 2, wobei das Gellangummi deacyliertes Gummi ist.

18. Tablette nach Anspruch 4, wobei das Arzneimittel aus der Gruppe, bestehend aus Antihistaminika, Antitussiva, Dekongestantien und Kombinationen davon, ausgewählt worden ist.

19. Tablette nach Anspruch 10, wobei das Arzneimittel aus der Gruppe, bestehend aus Acetaminophen, Acetylsalicylsäure, Celecoxib und Ibuprofen, ausgewählt worden ist.

## Revendications

1. Comprimé pharmaceutique comprenant un noyau et un enrobage y adhérant, comprimé dans lequel (a) le noyau comprend des particules solides d'un colorant hydrosoluble distribuées dans une matrice ; et (b) l'enrobage comprend de la gomme gellane.

2. Comprimé selon la revendication 1, ayant une surface d'apparence mouchetée.

3. Comprimé selon la revendication 1, dans lequel le noyau comprend un médicament en une quantité thérapeutiquement et/ou prophylactiquement efficace.

4. Comprimé selon la revendication 3, dans lequel le médicament est sélectionné dans le groupe consistant en les inhibiteurs de l'ACE ; agonistes α-adrénergiques ; agonistes β-adrénergiques ; bloqueurs α-adrénergiques ; bloqueurs β-adrénergiques ; médicaments dissuasifs contre la consommation d'alcool ; inhibiteurs de l'aldose réductase ; antagonistes de l'aldostérone ; acides aminés ; anabolisants ; analgésiques (tant narcotiques que non-narcotiques) ; anesthésiques ; anorexigènes ; antiacides ; anthelmintiques ; agents anti-acnéiques ; antiallergiques ; antiandrogènes ; agents antiangineux ; agents antianxiété ; antiarythmiques ; antiasthmatiques ; agents antibactériens et antibiotiques ; agents antialopécie et anticalvitie ; antiamibes ; anticorps ; médicaments anticholinergiques ; anticoagulants et fluidifiants du sang ; médicaments anticolite ; anticonvulsivants ; médicaments anticystite ; antidépresseurs ; agents antidiabétiques ; antidiarrhéiques ; antidiurétiques ; antidotes ; antiémétiques ; anti-oestrogènes ; antiflatulants ; agents antifongiques ; antigènes ; agents antiglaucome ; antihistaminiques ; antihyperactifs ; antihyperlipoprotéinémiques ; antihyper-tenseurs ; agents antihyperthyroïdiques ; antihypotenseurs ; agents antihypothyroïdiques ; anti-infectieux ; anti-inflammatoires (tant stéroïdiens que non-stéroïdiens) ; agents antimalaria ; agents antimigraineux ; anti-néoplasiques ; agents antiobésité ; agents anti-parkinsoniens et antidyskinésiques ; agents antipneumonie ; agents antiprotozoaires ; antiprurigineux ; anti-psoriasiques ; antipsychotiques ; antipyrétiques ; antirhumatismaux ; agents antisécrétoires ; médicaments antichoc ; antispasmodiques ; antithrombotiques ; agents antitumeur ; antitussifs ; antiulcéreux ; agents antiviraux ; anxiolytiques ; protéines de phase aiguë ; densifiants osseux ; bronchodilatateurs ; bloqueurs de canaux calciques ; inhibiteurs de l'anhydrase carbonique ; cardiotoniques et stimulants cardiaques ; chimio-thérapeutiques ; cholérétiques ; cholinergiques ; médicaments contre le syndrome de fatigue chronique ; stimulants du SNC ; coagulants ; contraceptifs ; médicaments contre la fibrose kystique ; décongestionnants ; diurétiques ; agonistes de récepteurs de la dopamine ; antagonistes de récepteurs de la dopamine ; enzymes ; oestrogènes ; expectorants ; médicaments contre l'hyperactivité gastrique ; glucocorticoïdes ; hémostatiques ; inhibiteurs de la HMG CoA réductase ; hormones ; hypnotiques ; immuno-modulateurs ; immunosuppresseurs ; laxatifs ; médicaments contre les maladies orales et périodontales ; myotiques ; inhibiteurs de la monoamine oxydase ; mucolytiques ; médicaments contre la sclérose en plaques ; myorelaxants ; mydriatiques ; antagonistes narcotiques ; antagonistes de récepteurs du NMDA ; oligonucléotides ; médicaments ophtalmiques ; oxytociques ; peptides, polypeptides et protéines ; polysaccharides ; progestogènes ; prostaglandines ; inhibiteurs de protéase ; stimulants respiratoires ; sédatifs ; inhibiteurs de la capture de la sérotonine ; hormones sexuelles ; médicaments pour cesser de fumer ; relaxants des muscles lisses ; stimulants des muscles lisses ; thrombolytiques ; tranquilisants ; acidifiants urinaires ; médicaments contre l'incontinence urinaire ; vasodilatateurs ; vasoprotecteurs ; et les combinaisons de ceux-ci.

5. Comprimé selon la revendication 3, dans lequel le médicament est un médicament pour cesser de fumer.

6. Comprimé selon la revendication 3, dans lequel le médicament est un médicament antibactérien.

7. Comprimé selon la revendication 3, dans lequel le médicament est un médicament antimigraineux.

8. Comprimé selon la revendication 3, dans lequel le médicament est utile pour le traitement ou la prévention d'un trouble ophtalmique.

9. Comprimé selon la revendication 3, dans lequel le médicament est un agent analgésique, antipyrétique ou anti-inflammatoire.

10. Comprimé selon la revendication 9, dans lequel l'agent analgésique, antipyrétique ou anti-inflammatoire est sélectionné dans le groupe consistant en l'acéclofénac, l'acémétacine, l'acide ε-acétamidocaproïque, l'acétaminophène, l'acétaminosalol, l'acétanilide, l'acide acétyl-salicylique, la S-adénosylméthionine, l'alclofénac, l'alclométasone, l'alfentanil, l'algestone, l'allylprodine, l'alminoprofène, l'aloxiprine, l'alphaprodine, le bis (acétylsalicylate) d'aluminium, l'amcinonide, l'amfénac, l'aminochlorthénoxazine, l'acide 3-amino-4-hydroxybutyrique, la 2-amino-4-picoline, l'aminopropylon, l'aminopyrine, l'amixétrine, le salicylate d'ammonium, l'ampiroxicam, l'amtolmétine guacil, l'aniléridine, l'antipyrine, l'antrafénine, l'apazone, la béclométhasone, le bendazac, le bénorylate, le benoxaprofène, le benzpipérylon, la benzydamine, la benzylmorphine, le bermoprofène, la bétaméthasone, le bézitramide, l'α-bisabolol, le bromfénac, le *p*-bromoacétanilide, l'acétate de l'acide 5-bromosalicylique, la bromosaligénine, la bucétine, l'acide bucloxique, le bucolome, le budésonide, le bufexamac, la bumadizone, la buprénorphine, la butacétine, le butibufène, le butorphanol, la carbamazépine, le carbiphène, le carprofène, le carsalam, le célécoxib, le chlorobutanol, la chloroprednisone, la chlorthénoxazine, le salicylate de choline, le cinchophène, la cinmétacine, le ciramadol, le clidanac, le clobétasol, la clocortolone, la clométacine, le clonitazène, la clonixine, le clopirac, le cloprednol, le clou de girofle, la codéine, le bromure de méthylcodéïne, le phosphate de codéine, le sulfate de codéine, la cortisone, le cortivazol, le cropropamide, le crotéthamide, le déflazacort, la désomorphine, le désonide, la désoximétasone, la dexaméthasone, le dexoxadrol, le dextromoramide, la dézocine, le diampromide, le diclofénac, le difénamizole, le difenpiramide, la diflorasone, la diflucortolone, le diflunisal, le difluprednate, la dihydrocodéine, l'énolacétate de dihydrocodéinone, la dihydromorphine, l'acétylsalicylate de dihydroxyaluminium, le diménoxadol, le dimépheptanol, le diméthylthiambutène, le butyrate de dioxaphétyle, la dipipanone, le diprocétyle, la dipyrone, le ditazole, le droxicam, l'émorfazone, l'acide enfénamique, l'énoxolone, l'épirizole, l'eptazocine, l'étersalate, l'éthenzamide, l'éthoheptazine, l'éthoxazène, l'éthylméthylthiambutène, l'éthylmorphine, l'étodolac, l'étofénamate, l'étonitazène, l'étoricoxib, l'eugénol, le felbinac, le fenbufène, l'acide fenclozique, le fendosal, le fénoprofène, le fentanyle, le fentiazac, le fépradinol, la féprazone, la floctafénine, le fluazacort, le flucloronide, l'acide flufénamique, la fluméthasone, le flunisolide, la flunixine, le flunoxaprofène, l'acétonide de fluocinolone, le fluocinonide, la butylfluocortine, la fluocortolone, la fluorésone, la fluorométholone, la flupérolone, la flupirtine, le fluprednidène, la fluprednisolone, la fluproquazone, le flurandrénolide, le flurbiprofène, le formocortal, le fosfosal, l'acide gentisique, la glafénine, la glucamétacine, le salicylate de glycol, le guaiazulène, l'halcinonide, l'halométasone, l'haloprednone, l'hydrocodone, l'hydrocortamate, l'hydrocortisone, l'hydromorphone, l'hydroxypéthidine, l'ibufénac, l'ibuprofène, l'ibuproxam, le salicylate d'imidazole, l'indométhacine, l'indoprofène, l'isofézolac, l'isoladol, l'isométhadone, l'isonixine, l'isoxépac, l'isoxicam, la kétobémidone, le kétoprofène, le kétorolac, le *p*-lactophénétide, la léfétamine, le lévorphanol, le lofentanil, le lonazolac, le lornoxicam, le loxoprofène, l'acétylsalicylate de lysine, la maziprédone, l'acide méclofénamique, la médrysone, l'acide méfénamique, ' la mépéridine, la meprednisone, le meptazinol, la mésalamine, la métazocine, la méthadone, la méthotriméprazine, la méthylprednisolone, l'acide métiazinique, la métofoline, le métopon, la mofébutazone, le mofézolac, la morazone, la morphine, le chlorhydrate de morphine, le sulfate de morphine, la salicylate de morphine, la myrophine, la nabumétone, la nalbuphine, le salicylate de 1-naphtyle, le naproxène, la narcéine, le néfopam, la Nicomorphine, la Nifénazone, l'acide niflumique, le nimésulide, le 5'-nitro-2'-propoxyacétanilide, le norlevorphanol, la norméthadone, la normorphine, la norpipanone, l'olsalazine, l'opium, l'oxacéprol, l'oxamétacine, l'oxaprozine, l'oxycodone, l'oxymorphone, l'oxyphenbutazone, le papaveretum, la paraméthasone, la paranyline, le parécoxib, le parsalmide, la pentazocine, le périsoxal, la phénacétine, la phénadoxone, la phénazocine, le chlorhydrate de phénazopyridine, le phénocolle, la phénopéridine, la phénopyrazone, l'acétylsalicylate de phényle, la phénylbutazone, le salicylate de phényle, le phényramidol, le pikétoprofène, la piminodine, la pipébuzone, la pipérylone, le piprofène, le pirazolac, le piritramide, le piroxicam, le pranoprofène, le prednicarbate, la prednisolone, la prednisone, le prednival, le prednylidène, la proglumétacine, la proheptazine, le promédol, le propacétamol, le propiram, le propoxyphène, la propyphénazone, la proquazone, l'acide protizinique, le proxazole, la ramifénazone, le rémifentanil, le méthylsulfate de rimazolium, le rofécoxib, le salacétamide, la salicine, le salicylamide, l'acide salicylamide-o-acétique, l'acide salicylique, l'acide salicylsulfurique, le salsalate, la salvérine, le simétride, le sufentanil, la sulfasalazine, le sulindac, la superoxyde dismutase, le suprofène, la suxibuzone, le talniflumate, le ténidap, le ténoxicam, le térofénamate, la tétrandrine, la thiazolinobutazone, l'acide tiaprofénique, le tiaramide, la tilidine, la tinoridine, le tixocortol, l'acide tolfénamique, la tolmétine, le tramadol, la triamcinolone, la tropésine, le valdécoxib, le viminol, la xenbucine, le ximoprofène, le zaltoprofène et le zomépirac.

11. Comprimé selon la revendication 3, dans lequel le médicament est un agent utile dans le traitement et/ou la prévention de la dysfonction sexuelle.

12. Comprimé selon l'une quelconque des revendications précédentes, qui convient à une utilisation perorale ou intraorale.

13. Comprimé selon l'une quelconque des revendications précédentes, dans lequel l'enrobage est présent en une quantité représentant un gain de poids de 0,1 % à 5 %.

14. Comprimé selon l'une quelconque des revendications précédentes, dans lequel la gomme gellane constitue de 25 % à 100 % en poids de l'enrobage.

15. Comprimé selon l'une quelconque des revendications précédentes, dans lequel l'enrobage comprend, en outre, au moins un excipient additionnel sélectionné dans le groupe consistant en les agents tampons, les plastifiants et les agents dispersants et émulsifiants.

16. Comprimé selon la revendication 1, dans lequel l'enrobage est appliqué par pulvérisation.

17. Comprimé selon la revendication 2, dans lequel la gomme gellane est déacylée.

18. Comprimé selon la revendication 4, dans lequel le médicament est sélectionné dans le groupe consistant en les antihistaminiques ; antitussifs ; décongestionnants ; et les combinaisons de ceux-ci.

19. Comprimé selon la revendication 10, dans lequel le médicament est sélectionné dans le groupe consistant en l'acétaminophène, l'acide acétyl-salicylique, le célécoxib et l'ibuprofène.
